# EUROPÄISCHE PATENTANMELDUNG

(11) **EP 3 858 282 A1**
(43) Veröffentlichungstag der Anmeldung: **04.08.2021**
(21) Anmeldenummer: 21154232.9
(22) Anmeldetag: 29.01.2021
(51) Int. Cl.: A61B 34/20, A61N 5/10

(54) **VERFAHREN ZUR VORBEREITUNG EINER BRACHYTHERAPIE UND BRACHYTHERAPIESYSTEM**

(30) Priorität: 30.01.2020 DE 102020102213
(71) Anmelder: Carl Zeiss Meditec AG, 07745 Jena (DE)
(72) Erfinder: NEIS, Valentin, 89075 Ulm (DE); NEIS, Friederike, 89075 Ulm (DE); WEIGAND, Frank, 89522 Heidenheim (DE)
(74) Vertreter: Diehl & Partner

(57) **Zusammenfassung**

Die Erfindung betrifft ein Verfahren zur Vorbereitung einer Brachytherapie, ein Brachytherapieverfahren und ein Brachytherapiesystem. Dabei kommen ein auf eine Strahlungsquelle (1) aufsetzbarer Applikator (3) und eine Applikatorführung (5), welche in einen zu bestrahlenden Gegenstand (31) einzuführen ist, zum Einsatz, wobei die Applikatorführung (5) so ausgebildet ist, dass der Applikator (3) in die Applikatorführung (5) eingeführt werden kann. Verfahrensgemäß werden eine Position und Orientierung der Applikatorführung (5) durch ein Navigationssystem erfasst, eine Position und Orientierung eines Einführungsassistenz-Grafikobjekts (43) in einer grafischen Darstellung (47) basierend auf der erfassten Position und Orientierung der Applikatorführung (5) durch eine Datenverarbeitungsvorrichtung bestimmt und das Einführungsassistenz-Grafikobjekt (43) an der bestimmten Position in der bestimmten Orientierung in der grafischen Darstellung (47) durch eine Anzeigevorrichtung (29) angezeigt. Das Einführungsassistenz-Grafikobjekt (43) ist durch seine Gestalt und seine Darstellung in der grafischen Darstellung (47) dazu geeignet ist, das Einführen des Applikators (3) in die Applikatorführung (5) durch einen Betrachter zu erleichtern.

## Beschreibung

Die vorliegende Erfindung betrifft ein Verfahren zur Vorbereitung einer Brachytherapie, ein Brachytherapieverfahren und ein Brachytherapiesystem. Insbesondere betrifft die vorliegende Erfindung ein Hilfsmittel zum Einführen eines Applikators, der auf eine Strahlungsquelle aufgesetzt werden kann, in eine Applikatorführung, welche in einen zu bestrahlenden Gegenstand eingeführt werden kann.

Im Bereich der Brachytherapie unter Verwendung von Röntgenstrahlung existieren Therapien, bei denen Röntgenstrahlung im Inneren eines Gegenstands, insbesondere eines menschlichen oder tierischen Körpers, in der Nähe eines zu bestrahlenden Bereichs, insbesondere in der Nähe von zu therapierendem Gewebe, erzeugt werden soll. Die Röntgenstrahlung kann hierbei durch ein Bestrahlungsgerät bereitgestellt werden. Ein beispielhaftes Bestrahlungsgerät umfasst ein Teilchenstrahlsystem, welches einen hochenergetischen Teilchenstrahl erzeugen kann. Der Teilchenstrahl wird durch ein einige Zentimeter langes Rohr des Bestrahlungsgeräts auf ein Röntgen-Material gerichtet, welches am Ende des Rohres angeordnet ist. Durch Wechselwirkung des Teilchenstrahls mit dem Röntgen-Material wird von dem Röntgen-Material Röntgenstrahlung emittiert. Diese Röntgenstrahlung tritt durch das Rohr aus und trifft auf den zu bestrahlenden Bereich.

Damit die von dem Röntgen-Material an der Spitze des Rohres erzeugte Röntgenstrahlung im Inneren eines Gegenstands appliziert werden kann, wird das Rohr in den Gegenstand eingeführt. Hierzu ist das Rohr von einem Applikator umgeben, welcher einerseits eine sterile Barriere darstellt und andererseits das Rohr schützt.

Vor dem Einführen des Rohres in den Gegenstand wird in den Gegenstand eine Applikatorführung eingeführt. Die Applikatorführung umfasst beispielsweise einen rohrförmigen Abschnitt, der an einem Ende eine Öffnung zum Einführen des Applikators aufweist. Nachdem die Applikatorführung in den Gegenstand eingeführt wurde, kann das mit dem Applikator versehene Bestrahlungsgerät durch die Öffnung des rohrförmigen Abschnitts in die Applikatorführung eingeführt werden. Auf diese Weise wird das Röntgen-Material im Inneren des Gegenstands angeordnet. Daraufhin kann das Röntgen-Material durch Wechselwirkung mit dem Teilchenstrahl die Röntgenstrahlung im Inneren des Gegenstands erzeugen.

Bei dieser Vorgehensweise besteht jedoch das Problem, dass von der Applikatorführung nur noch ein kurzer Abschnitt zu sehen ist, sobald die Applikatorführung in den Gegenstand eingeführt wurde. Es kann daher schwierig sein, die Orientierung der Applikatorführung mit dem Auge zu erkennen. Beim Einführen des Applikators in die Applikatorführung muss der Applikator jedoch relativ genau zu der Applikatorführung ausgerichtet sein, damit der Applikator und das darin angeordnete Rohr des Bestrahlungsgeräts beim Einführen nicht verbogen werden. Wenn das Rohr verbogen ist, ist die Erzeugung der Röntgenstrahlung gestört.

Es ist daher eine Aufgabe der vorliegenden Erfindung, ein Hilfsmittel bereitzustellen, welches das Einführen des Applikators in die Applikatorführung erleichtert.

Ein erster Aspekt der Erfindung betrifft ein Verfahren zur Vorbereitung einer Brachytherapie, wobei das Verfahren umfasst: Bereitstellen eines Applikators und einer Applikatorführung, wobei die Applikatorführung so ausgebildet ist, dass der Applikator in die Applikatorführung eingeführt werden kann und die Applikatorführung die Bewegung des Applikators beim Einführen des Applikators in die Applikatorführung führt; Erfassen einer Position und Orientierung der Applikatorführung durch ein Navigationssystem; Bestimmen einer Position und Orientierung eines Einführungsassistenz-Grafikobjekts in einer grafischen Darstellung basierend auf der erfassten Position und Orientierung der Applikatorführung durch eine Datenverarbeitungsvorrichtung; Anzeigen des Einführungsassistenz-Grafikobjekts in der grafischen Darstellung an der bestimmten Position in der bestimmten Orientierung durch eine Anzeigevorrichtung, wobei das Einführungsassistenz-Grafikobjekt durch seine Gestalt dazu geeignet ist, das Einführen des Applikators in die Applikatorführung durch einen Betrachter zu erleichtern.

Die Position und Orientierung des Einführungsassistenz-Grafikobjekts können beispielsweise durch Berechnung durch die Datenverarbeitungsvorrichtung bestimmt werden, wobei die Berechnung die erfasste Position und Orientierung der Applikatorführung verwendet. Da die Position und die Orientierung des Einführungsassistenz-Grafikobjekts in Abhängigkeit der erfassten Position der (realweltlichen) Applikatorführung bestimmt werden, ändern sich die Position und Orientierung des Einführungsassistenz-Grafikobjekts, wenn sich die Position und Orientierung der (realweltlichen) Applikatorführung ändert.

Hierin bezeichnet ein Grafikobjekt ein virtuelles Objekt, das nur durch seine visuelle Darstellung einem menschlichen Beobachter sichtbar wird. Ein Grafikobjekt hat keinen massebehafteten Körper. Ein Grafikobjekt hat eine Gestalt, die eine Gestalt eines realweltlichen Objekts nachbilden kann. Beispielsweise kann das Einführungsassistenz-Grafikobjekt eine Gestalt haben, die die Gestalt des Applikators nachbildet. Die Gestalt eines realweltlichen Objekts ist durch seine körperliche Form gegeben.

Einzelne oder alle Schritte des Verfahrens können wiederholt werden. Beispielsweise können das Erfassen der Position und Orientierung der Applikatorführung, das Bestimmen der Position und Orientierung des Einführungsassistenz-Grafikobjekt in der grafischen Darstellung und das Anzeigen des Einführungsassistenz-Grafikobjekts wiederholt durchgeführt werden. Hierdurch wirkt sich eine Änderung der Position und Orientierung der Applikatorführung auf die Darstellung des Einführungsassistenz-Grafikobjekts dahingehend aus, dass das Einführungsassistenz-Grafikobjekt an der neu bestimmten Position in der neu bestimmten Orientierung dargestellt wird.

Das obige Verfahren bewirkt, dass das Einführungsassistenz-Grafikobjekt an einer bestimmten Position in einer bestimmen Orientierung dargestellt wird und somit durch einen Betrachter, der den Applikator in die Applikatorführung einführen möchte, visuell betrachtet werden kann. Das Einführungsassistenz-Grafikobjekt ist dem Betrachter durch seine Gestalt und seine bestimmte Position und Orientierung eine Hilfe beim Einführen des Applikators in die Applikatorführung, da es dem Betrachter eine Anleitung oder Anweisung zum Bewegen des Applikators bereitstellt.

Ein zweiter Aspekt der Erfindung betrifft ein Brachytherapieverfahren. Das Brachytherapieverfahren umfasst die Schritte des Verfahrens zur Vorbereitung einer Brachytherapie gemäß dem ersten Aspekt, wobei vor dem Erfassen der Position und Orientierung der Applikatorführung die Applikatorführung in einen Gegenstand, insbesondere einen menschlichen oder tierischen Körper, eingeführt wird und wobei bei dem Erfassen der Position und Orientierung der Applikatorführung die Position und die Orientierung der in den Gegenstand eingeführten Applikatorführung erfasst werden. Das Brachytherapieverfahren kann insbesondere weiter umfassen: Einführen des Applikators in die Applikatorführung unter Verwendung des dargestellten Einführungsassistenz-Grafikobjekts; und/oder Erzeugen von Röntgenstrahlung im Inneren des in die Applikatorführung eingeführten Applikators.

Ein dritter Aspekt der Erfindung betrifft ein System, insbesondere ein Brachytherapiesystem, welches so konfiguriert ist, dass damit die hierin beschriebenen Verfahren durchgeführt werden können. Das System umfasst insbesondere einen Applikator, eine Applikatorführung, ein Navigationssystem, eine Datenverarbeitungsvorrichtung und eine Anzeigevorrichtung.

Ausführungsformen der Erfindung werden nachfolgend anhand von Figuren näher erläutert. Hierbei zeigt:
- Figur 1: eine schematische Darstellung eines Bestrahlungsgeräts, eines Applikators und einer Applikatorführung;
- Figur 2: eine schematische Darstellung eines Brachytherapiesystems;
- Figur 3: eine schematische Darstellung einer grafischen Darstellung mit Objekten der erweiterten Realität zur Anzeige durch eine Anzeigevorrichtung;
- Figur 4: eine schematische Darstellung einer weiteren grafischen Darstellung mit Objekten der erweiterten Realität zur Anzeige durch eine Anzeigevorrichtung;
- Figur 5: eine schematische Darstellung einer weiteren grafischen Darstellung mit Objekten der erweiterten Realität zur Anzeige durch eine Anzeigevorrichtung; und
- Figur 6: eine schematische Darstellung einer grafischen Darstellung mit Objekten der virtuellen Realität zur Anzeige durch eine Anzeigevorrichtung.

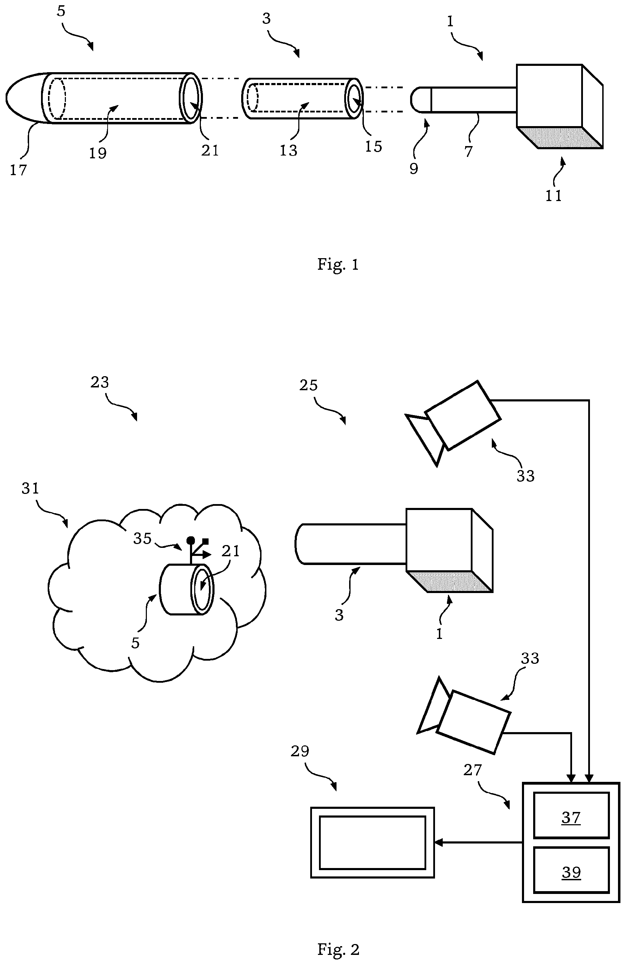

Figur 1 zeigt in schematischer Darstellung ein Bestrahlungsgerät 1, einen Applikator 3 und eine Applikatorführung 5.

Das Bestrahlungsgerät 1 umfasst ein einige Zentimeter langes Rohr 7, an dessen einem Ende ein Röntgen-Material 9 angeordnet ist. Das Bestrahlungsgerät 1 umfasst ferner ein Teilchenstrahlsystem 11, welches dazu konfiguriert ist, einen Teilchenstrahl zu erzeugen und durch das Rohr 7 auf das Röntgen-Material 9 zu richten. Hierdurch wird Röntgenstrahlung durch Wechselwirkung des Teilchenstrahls mit dem Röntgen-Material 9 erzeugt. Das Bestrahlungsgerät 1 kann von einem Stativ getragen werden, was die Handhabung (Bewegung und Orientierung) des Bestrahlungsgeräts 1 für einen Nutzer erleichtert. Das Bestrahlungsgerät 1 kann anders konfiguriert sein. Die Erfindung ist auf alle Bestrahlungsgeräte anwendbar, die das Rohr 7 oder einen funktionell ähnlichen länglichen Abschnitt aufweisen.

Der Applikator 3 hat in dem in Figur 1 gezeigten Beispiel eine zylinderförmige Gestalt. Der Applikator 3 kann jedoch auch andere Gestalten aufweisen. Der Applikator 3 weist einen auf die Gestalt des Rohres 7 des Bestrahlungsgeräts 1 angepassten Hohlraum 13 im Inneren des Applikators 3 auf. Der Hohlraum 13 ist durch gestrichelte Linien schematisch dargestellt. Der Hohlraum 13 erstreckt sich bis zu einer Öffnung 15 des Applikators 3. Die Öffnung 15 und der daran anschließende Hohlraum 13 sind so gestaltet, dass das Rohr 7 des Bestrahlungsgeräts 1 wenigstens teilweise durch die Öffnung 15 in den Hohlraum 13 des Applikators 3 eingeführt werden kann. Das Rohr 7 kann in der Praxis einige Zentimeter in den Hohlraum 13 eingeführt werden. Die Gestalt des Hohlraumes 13 des Applikators 3 bewirkt eine Führung des Rohres 7 beim Einführen des Rohres 7 in den Hohlraum 13 des Applikators 3. Wenn der Applikator 3 auf diese Weise auf das Bestrahlungsgerät 1 aufgesetzt ist, schützt der Applikator 3 das Rohr 7 des Bestrahlungsgeräts 1 vor äußeren Einflüssen.

Die Applikatorführung 5 weist in dem in Figur 1 gezeigten Beispiel eine im Wesentlichen zylinderförmige Gestalt mit einem abgerundeten Ende 17 auf. Das abgerundete Ende 17 dient der einfachen Einführung der Applikatorführung 5 in einen Gegenstand. Die Applikatorführung 5 weist im Inneren einen Hohlraum 19 auf, der sich bis zu einer Öffnung 21 erstreckt. Die Öffnung 21 und der Hohlraum 19 der Applikatorführung 5 sind so gestaltet, dass der Applikator 3 wenigstens teilweise durch die Öffnung 21 in den Hohlraum 19 der Applikatorführung 5 eingeführt werden kann. Die Applikatorführung 5 kann in der Praxis einige Zentimeter in den Gegenstand 31 eingeführt werden. Die Gestalt des Hohlraumes 19 der Applikatorführung 5 bewirkt eine Führung des Applikators 3 beim Einführen des Applikators 3 in den Hohlraum 19 der Applikatorführung 5.

Figur 2 zeigt in schematischer Darstellung ein Brachytherapiesystem 23, mit welchem die hierin beschriebenen Verfahren durchgeführt werden können. Das Brachytherapiesystem 23 umfasst den Applikator 3, die Applikatorführung 5, ein Navigationssystem 25, eine Datenverarbeitungsvorrichtung 27 und eine Anzeigevorrichtung 29.

In der in Figur 2 dargestellten Situation ist das Bestrahlungsgerät 1 in den Applikator 3 eingeführt. Ferner ist die Applikatorführung 5 in einen Gegenstand 31 eingeführt. Lediglich ein kurzer Abschnitt der Applikatorführung 5, welcher die Öffnung 21 aufweist, ragt noch aus dem Gegenstand 31 heraus. Dies ist eine typische Situation während einem Brachytherapieverfahren. Hierbei wird das Problem deutlich, dass die Orientierung der Applikatorführung 5 schwer zu erkennen ist, da ein Großteil der Applikatorführung 5 in dem Gegenstand 31 angeordnet ist und daher nicht sichtbar ist. Es ist daher schwierig, den Applikator 3 in die Applikatorführung 5 einzuführen, da die Orientierung des Applikators 3 auf die Orientierung der Applikatorführung 5 auszurichten ist, was jedoch schwierig ist, wenn die Orientierung der Applikatorführung 5 selbst schwer zu erkennen ist.

Dieses Problem wird durch das Brachytherapiesystem 23 wie nachfolgend erläutert gelöst.

Das Navigationssystem 25 ist dazu konfiguriert, die Position und die Orientierung der Applikatorführung 5 zu erfassen. Das Navigationssystem 25 kann auf diverse Weisen konfiguriert sein. Das in den Figuren dargestellte und hierin beschriebene Navigationssystem 25 zeigt lediglich eine mögliche Konfiguration.

Das Navigationssystem 25 umfasst mehrere Kameras 33, welche dazu konfiguriert sind ein Bild bzw. ein Video der (in den Gegenstand 31 eingeführten) Applikatorführung 5 aufzunehmen. Die Kameras 33 können beispielsweise herkömmliche Farbbildkameras sein.

Das Navigationssystem 25 umfasst ferner eine Markierung 35. Die Markierung 35 ist in einer bekannten Position und Orientierung bezüglich der Applikatorführung 5 mit der Applikatorführung 5 verbindbar. Wenn die Markierung 35 in der bekannten Position und Orientierung bezüglich der Applikatorführung 5 mit der Applikatorführung 5 verbunden ist, so wie dies in Figur 2 gezeigt ist, ändert sich die Orientierung der Markierung 35 gemäß der Änderung der Orientierung der Applikatorführung 5. Wenn die Markierung 35 in der bekannten Position und Orientierung bezüglich der Applikatorführung 5 mit der Applikatorführung 5 verbunden ist, können die Position und Orientierung der Applikatorführung 5 basierend auf der Position und Orientierung der Markierung 35 bestimmt werden, da der räumliche Zusammenhang bekannt ist.

In dem in Figur 2 gezeigten Beispiel besteht die Markierung 35 aus drei Stäben, die zueinander jeweils orthogonal orientiert sind. An den Enden der Stäbe befinden sich eine Kugel, ein Würfel und eine Pyramide. Diese Konfiguration der Markierung 35 erlaubt es, die Position und Orientierung der Markierung 35 auf Grundlage der Bilder zu bestimmen, die von den Kameras 33 aufgenommen werden. Die in Zusammenhang mit Figur 2 erläuterte Konfiguration der Markierung 35 ist lediglich beispielhaft. Beispielsweise können statt der Kugel, dem Würfel und der Pyramide andere unterscheidbare Markierungselemente, beispielsweise drei gleiche Symbole mit unterschiedlichen Farben oder dergleichen, verwendet werden.

Das Navigationssystem 25 umfasst ferner einen Datenverarbeitungsabschnitt 37, welcher beispielsweise in der Datenverarbeitungsvorrichtung 27 bereitgestellt ist. Der Datenverarbeitungsabschnitt 37 für das Navigationssystem 25 empfängt die von den Kameras 33 aufgenommenen Bilder und bestimmt daraus die Position und Orientierung der Markierung 35 und daraus die Position und Orientierung der Applikatorführung 5. Die Bestimmung der Position und Orientierung der Applikatorführung 5 ist nicht auf das hierin beschrieben Beispiel beschränkt. Andere Techniken können hierzu verwendet werden.

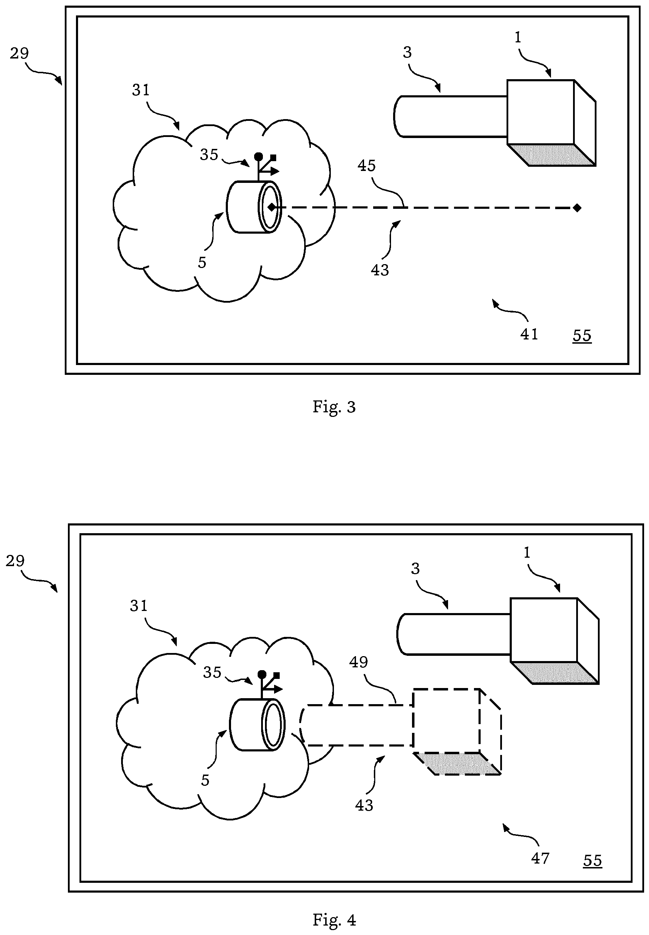

Basierend auf der erfassten Position und Orientierung der Applikatorführung 5 bestimmt ein Datenverarbeitungsabschnitt 39 der Datenverarbeitungsvorrichtung 27 eine Position und Orientierung eines Einführungsassistenz-Grafikobjekts in einer grafischen Darstellung, welche mittels der Anzeigevorrichtung 29 angezeigt wird. Dies wird nachfolgend mit Bezug zu den Figuren 3 und 4 näher erläutert.

Figur 3 zeigt eine schematische Darstellung einer grafischen Darstellung 41, die durch die Datenverarbeitungsvorrichtung 27 erzeugt und durch die Anzeigevorrichtung 29 visuell wahrnehmbar angezeigt wird. Die grafische Darstellung 41 kann eine Vielzahl von Grafikobjekten umfassen.

Die Anzeigevorrichtung 29 ist beispielsweise eine "augmented reality"-Brille (AR-Brille), welche einem Nutzer gleichzeitig die direkte unmittelbare Betrachtung einer realweltlichen Szene einerseits und die Betrachtung von virtuellen Objekten der erweiterten Realität andererseits ermöglicht. Die Anzeigevorrichtung 27 nach Art der AR-Brille weist einen Anzeigebereich 55 auf, der von einem Betrachter betrachtet werden kann. Auf dem Anzeigebereich 55 wird die grafische Darstellung 41 visuell wahrnehmbar angezeigt. Der Anzeigebereich 55 ist im Übrigen transparent, sodass in den Bereichen des Anzeigebereichs 55, an denen die grafische Darstellung 41 keine Grafikobjekte aufweist, die realweltliche Szene unmittelbar betrachtet werden kann. Die in der grafischen Darstellung 41 enthaltenen Grafikobjekte können daher im Umfeld der realweltlichen Szene betrachtet werden. Somit wird durch die AR-Brille die realweltliche Szene mit Grafikobjekten der grafischen Darstellung 41 erweitert, die in der realweltlichen Szene selbst nicht enthalten sind.

Figur 3 zeigt in schematischer Darstellung die grafische Darstellung 41 im Umfeld einer realweltlichen Szene durch eine Anzeigevorrichtung 27 nach Art der AR-Brille.

Die realweltliche Szene umfasst das Bestrahlungsgerät 1, den Applikator 3, die Applikatorführung 5 mit Markierung 35 und den Gegenstand 31.

Die grafische Darstellung 41 umfasst ein Einführungsassistenz-Grafikobjekt 43. In dem in Figur 3 gezeigten Beispiel umfasst das Einführungsassistenz-Grafikobjekt 43 eine Leitlinie 45. Die Leitlinie 45 wird durch eine gestrichelte Linie mit Rauten an den Enden dargestellt. Die grafische Darstellung von Leitlinien ist darauf nicht beschränkt. Die Anzeige der grafischen Darstellung 41 mit der Leitlinie 45 durch die Anzeigevorrichtung 29 im Umfeld der realweltlichen Szene erleichtert einem Betrachter die Einführung des Applikators 3 in die Applikatorführung 5.

Durch den Datenverarbeitungsabschnitt 39 werden die Position und Orientierung des Einführungsassistenz-Grafikobjekts 43 bzw. die Position und Orientierung der Leitlinie 45 in der grafischen Darstellung 41 beispielsweise so bestimmt, dass das Einführungsassistenz-Grafikobjekt 43 bzw. die Leitlinie 45 in Verlängerung des Hohlraums 19 der Applikatorführung 5 erscheint. Das bedeutet beispielsweise, dass die Leitlinie 45 so angezeigt wird, als wäre sie im realweltlichen Raum parallel zu dem zylinderförmigen Hohlraum 19 der Applikatorführung 5 orientiert. Die Leitlinie 45 kann dabei in der grafischen Darstellung 41 so positioniert sein, dass sie so erscheint, als würde die Leitlinie 45 die Öffnung 21 der Applikatorführung 5 durchsetzen.

Um die Position und Orientierung des Einführungsassistenz-Grafikobjekts 43 bzw. der Leitlinie 45 in der grafischen Darstellung 41 an der realweltlichen Position und Orientierung der Applikatorführung 5 ausrichten zu können, bestimmt der Datenverarbeitungsabschnitt 39 die Position und Orientierung des Einführungsassistenz-Grafikobjekts 43 bzw. der Leitlinie 45 auf Basis der zuvor mittels des Navigationssystems 25 erfassten Position und Orientierung der Applikatorführung 5.

Bei der Anzeige durch eine AR-Brille hängt die grafische Darstellung bzw. die Position und Orientierung der Grafikobjekte der grafischen Darstellung von der Position und Richtung ab, aus welcher der Betrachter die realweltliche Szene betrachtet. Um dies berücksichtigen zu können, kann das Navigationssystem 25 ferner dazu konfiguriert sein, die Position und Orientierung der Anzeigevorrichtung 29 zu erfassen, und die Datenverarbeitungsvorrichtung 27 kann dazu konfiguriert sein, die Position und Orientierung des Einführungsassistenz-Grafikobjekts 43 in der grafischen Darstellung 41 ferner basierend auf der erfassten Position und Orientierung der Anzeigevorrichtung 29 zu bestimmen.

Die Datenverarbeitungsvorrichtung 27 veranlasst die Anzeigevorrichtung 29, das Einführungsassistenz-Grafikobjekt 43 an der bestimmten Position in der bestimmten Orientierung in der grafischen Darstellung 41 anzuzeigen. Durch seine Gestalt und seine Darstellung an der bestimmten Position in der bestimmten Orientierung ist das Einführungsassistenz-Grafikobjekt 43 dazu geeignet, das Einführen des Applikators 3 in die Applikatorführung 5 durch einen Betrachter zu erleichtern. Dies geschieht, indem der Betrachter die angezeigte grafische Darstellung 41 im Umfeld der realweltlichen Szene betrachtet und durch die Gestalt und die Position und Orientierung des dargestellten Einführungsassistenz-Grafikobjekts 43 eine visuelle Hilfe erhält, die die Positionierung und Orientierung des Applikators 3 vereinfacht.

Figur 4 zeigt in schematischer Darstellung eine weitere grafische Darstellung 47 im Umfeld einer realweltlichen Szene durch eine Anzeigevorrichtung 27 nach Art der AR-Brille. Die grafische Darstellung 47 wird von der Datenverarbeitungsvorrichtung 27 erzeugt und durch die Anzeigevorrichtung 29 angezeigt.

In der in Figur 4 gezeigten grafischen Darstellung 47 umfasst das Einführungsassistenz-Grafikobjekt 43 ein Grafikobjekt 49, welches die Gestalt des Applikators 3 (und des Bestrahlungsgeräts 1) wiedergibt. Der Datenverarbeitungsabschnitt 39 bestimmt die Position und Orientierung des Grafikobjekts 49 in der grafischen Darstellung 47 beispielsweise so, dass sie eine Zielposition und eine Zielorientierung für den Applikator 3 repräsentieren, die durch Bewegen des Applikators 3 erreicht werden soll, um den Applikator 3 in die Applikatorführung 5 einführen zu können. Beispielsweise werden die Position und Orientierung des Grafikobjekts 49 in der grafischen Darstellung 47 so bestimmt, dass das Grafikobjekt 49 in Verlängerung des Hohlraums 19 der Applikatorführung 5 erscheint. Zur Darstellung des Grafikobjekts 49 in Gestalt des Applikators 3 umfasst die Datenverarbeitungsvorrichtung 27 einen Datenspeicher, aus welchem Formdaten erhalten werden können, die die Gestalt des Applikators 3 repräsentieren. Formdaten repräsentieren die räumliche Gestalt eines realweltlichen Gegenstands. Diese Formdaten werden zur Darstellung des Grafikobjekts 49 bzw. zur Darstellung des Einführungsassistenz-Grafikobjekts 43 verwendet.

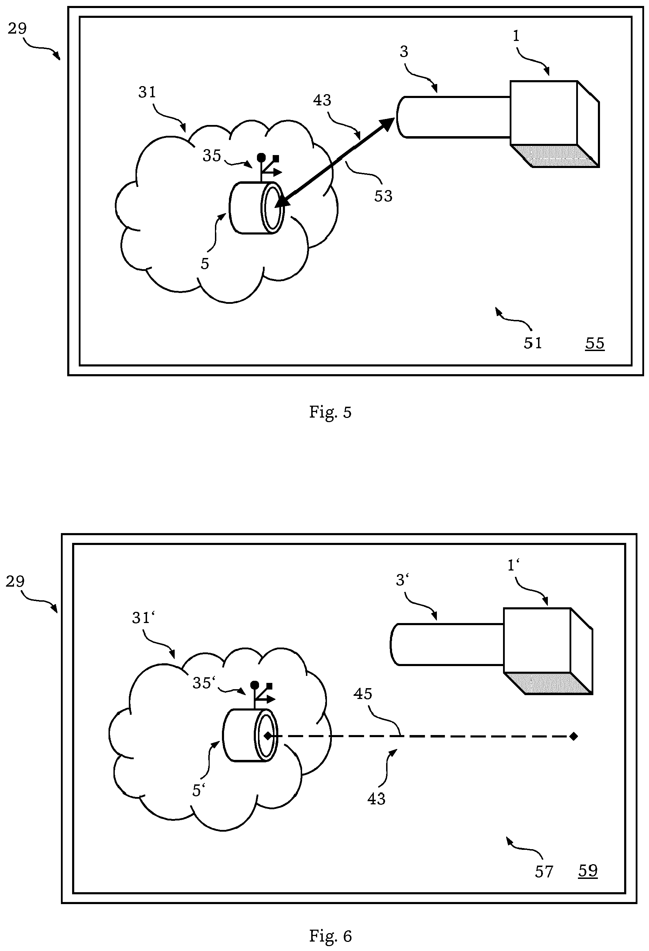

Eine weitere Ausführungsform wird mit Bezug zu Figur 5 beschrieben. Das Navigationssystem 25 kann ferner dazu konfiguriert sein, die Position und/oder Orientierung des Applikators 3 zu bestimmen. Die Technik zum Erfassen der Position und/oder Orientierung des Applikators 3 kann gleich sein wie die Technik zum Erfassen der Position und Orientierung der Applikatorführung 5. Alternativ kann die Position und/oder Orientierung des Applikators 3 mit einer anderen Technik durch das Navigationssystem 25 erfasst werden.

Der Datenverarbeitungsabschnitt 39 kann ferner dazu konfiguriert sein, basierend auf der erfassten Position und/oder Orientierung des Applikators 3 und auf der erfassten Position und/oder Orientierung der Applikatorführung 5 eine Hilfsgröße zu bestimmen. Die Hilfsgröße ist beispielsweise ein Abstand zwischen dem Applikator 3 und der Applikatorführung 5. Zum Bestimmen des Abstands zwischen dem Applikator 3 und der Applikatorführung 5 verwendet der Datenverarbeitungsabschnitt 39 beispielsweise die mittels des Navigationssystems 25 erfasste Position des Applikators 3 und Position der Applikatorführung 5 sowie Formdaten, die die Gestalt des Applikators 3 und die Gestalt der Applikatorführung 5 repräsentieren. Je nach Art der Hilfsgröße basiert das Bestimmen der Hilfsgröße auf der erfassten Position des Applikators 3 und/oder auf der erfassten Orientierung des Applikators 3 und/oder auf der erfassten Position der Applikatorführung 5 und/oder auf der erfassten Orientierung der Applikatorführung 5 und/oder Formdaten über den Applikator 3 und/oder Formdaten über die Applikatorführung 5.

Der Datenverarbeitungsabschnitt 39 ist ferner dazu konfiguriert, die Hilfsgröße als Teil des Einführungsassistenz-Grafikobjekts 43 darzustellen.

Figur 5 zeigt eine schematische Darstellung einer grafischen Darstellung 51 im Umfeld einer realweltlichen Szene durch eine Anzeigevorrichtung 27 nach Art der AR-Brille.

Die grafische Darstellung 51 wird durch die Datenverarbeitungsvorrichtung 27 erzeugt und durch die Anzeigevorrichtung 29 angezeigt. Die grafische Darstellung 51 umfasst ein Einführungsassistenz-Grafikobjekt 43, welches eine Hilfsgröße 53 umfasst. Die Hilfsgröße 53 ist durch einen Doppelpfeil dargestellt und repräsentiert den von dem Datenverarbeitungsabschnitt 39 bestimmten Abstand zwischen dem Applikator 3 und der Applikatorführung 5.

Mit Bezug zu den Figuren 3 bis 5 wurden Beispiele für grafische Darstellungen erläutert, die im Umfeld einer realweltlichen Szene durch eine Anzeigevorrichtung 27 nach Art der AR-Brille angezeigt werden. Die Art der Anzeige ist darauf nicht beschränkt. Nachfolgend wird mit Bezug zu Figur 6 ein Beispiel erläutert, bei welchem das Einführungsassistenz-Grafikobjekt 43 als Objekt der virtuellen Realität bzw. in einem Bild dargestellt wird.

Figur 6 zeigt eine schematische Darstellung einer grafischen Darstellung 57, welche von der Datenverarbeitungsvorrichtung 27 erzeugt und durch die Anzeigevorrichtung 29 angezeigt wird. Die Anzeigevorrichtung 29 ist beispielsweise eine "virtual reality"-Brille oder ein Monitor. Diese Geräte zeigen einem Betrachter ein Bild 59 an, welches die grafische Darstellung 57 umfasst. Der Bildbereich des Bildes 59 umfasst keine realweltlichen Objekte, sondern ausschließlich Objekte der virtuellen Realität. In diesem Sinne ist eine konventionelle Fotografie, also eine Aufnahme einer realweltlichen Szene und die visuell sichtbare Wiedergabe der Aufnahme, ein Bild, welches ausschließlich Objekte der virtuellen Realität darstellt.

Die grafische Darstellung 57 umfasst eine Vielzahl von Grafikobjekten, die realweltliche Objekte repräsentieren. Beispielsweise umfasst die grafische Darstellung 57 einen Bildbereich 1', welcher die Wiedergabe der Aufnahme des Bestrahlungsgeräts 1 repräsentiert, einen Bildbereich 3', welcher die Wiedergabe der Aufnahme des Applikators 3 repräsentiert, einen Bildbereich 5', welcher die Wiedergabe der Aufnahme der Applikatorführung 5 repräsentiert, und einen Bildbereich 31', welcher die Wiedergabe einer Aufnahme des Gegenstands 31 repräsentiert. Die Bildbereiche 1', 3', 5' und 31' sind Grafikobjekte der grafischen Darstellung 57, die realweltliche Objekte repräsentieren.

Die grafische Darstellung 57 umfasst ferner Grafikobjekte, die virtuelle Objekte repräsentieren. Die grafische Darstellung 57 umfasst beispielsweise das Einführungsassistenz-Grafikobjekt 43, welches der Aufnahme der realweltlichen Szene durch Bildbearbeitung durch den Datenverarbeitungsabschnitt 39 hinzugefügt wurde.

Durch seine Gestalt und seine Darstellung an der bestimmten Position in der bestimmten Orientierung ist das Einführungsassistenz-Grafikobjekt 43 dazu geeignet, das Einführen des Applikators 3 in die Applikatorführung 5 durch einen Betrachter zu erleichtern. Dies geschieht, indem der Betrachter die angezeigte grafische Darstellung 57 betrachtet und durch die Gestalt und die Position und Orientierung des dargestellten Einführungsassistenz-Grafikobjekts 43 eine visuelle Hilfe erhält, die die Positionierung und Orientierung des Applikators 3 vereinfacht.

## Patentansprüche

1. Verfahren zur Vorbereitung einer Brachytherapie, umfassend:
Bereitstellen eines Applikators (3) und einer Applikatorführung (5), wobei die Applikatorführung (5) so ausgebildet ist, dass der Applikator (3) in die Applikatorführung (5) eingeführt werden kann und die Applikatorführung (5) die Bewegung des Applikators (3) beim Einführen des Applikators (3) in die Applikatorführung (5) führt;
Erfassen einer Position und Orientierung der Applikatorführung (5) durch ein Navigationssystem (25);
Bestimmen einer Position und Orientierung eines Einführungsassistenz-Grafikobjekts (43) in einer grafischen Darstellung (41, 47, 51, 57) basierend auf der erfassten Position und Orientierung der Applikatorführung (5) durch eine Datenverarbeitungsvorrichtung (27);
Anzeigen des Einführungsassistenz-Grafikobjekts (43) an der bestimmten Position in der bestimmten Orientierung in der grafischen Darstellung (41, 47, 51, 57) durch eine Anzeigevorrichtung (29),
wobei das Einführungsassistenz-Grafikobjekt (43) durch seine Gestalt dazu geeignet ist, das Einführen des Applikators (3) in die Applikatorführung (5) durch einen Betrachter zu erleichtern.

2. Verfahren nach Anspruch 1, wobei das Einführungsassistenz-Grafikobjekt (43) ein Grafikobjekt (49) umfasst, welches die Gestalt des Applikators (3) wiedergibt.

3. Verfahren nach Anspruch 2, ferner umfassend:
Erhalten von Formdaten, die die Gestalt des Applikators (3) repräsentieren, aus einem Datenspeicher; und
Verwenden der Formdaten bei dem Darstellen des Einführungsassistenz-Grafikobjekts (43).

4. Verfahren einem der Ansprüche 1 bis 3, wobei das Einführungsassistenz-Grafikobjekt (43) mindestens eine Leitlinie (45) umfasst.

5. Verfahren einem der Ansprüche 1 bis 4, wobei die Position und die Orientierung des Einführungsassistenz-Grafikobjekts (43) an der erfassten Position und Orientierung der Applikatorführung (5) ausgerichtet sind.

6. Verfahren nach einem der Ansprüche 1 bis 5,
wobei die Applikatorführung (5) einen Hohlraum (19) umfasst, in welchen der Applikator (3) einzuführen ist, und
wobei die Position und Orientierung des Einführungsassistenz-Grafikobjekts (43) in der grafischen Darstellung (41, 47, 51, 57) so bestimmt werden, dass das Einführungsassistenz-Grafikobjekt (43) in Verlängerung des Hohlraums (19) erscheint.

7. Verfahren nach einem der Ansprüche 1 bis 6, wobei die Position und Orientierung des Einführungsassistenz-Grafikobjekts (43) so bestimmt werden, dass sie eine Zielposition und eine Zielorientierung für den Applikator (3) repräsentieren, die durch Bewegen des Applikators (3) erreicht werden soll, um den Applikator (3) in die Applikatorführung (5) einführen zu können.

8. Verfahren nach einem der Ansprüche 1 bis 7, ferner umfassend:
Erfassen einer Position und Orientierung des Applikators (3) durch das Navigationssystem (25);
Bestimmen einer Hilfsgröße (53), insbesondere eines Abstands zwischen dem Applikator (3) und der Applikatorführung (5), basierend auf der erfassten Position und/oder Orientierung des Applikators (3) und auf der erfassten Position und/oder Orientierung der Applikatorführung (5); und
Darstellen der Hilfsgröße (53) als Teil des Einführungsassistenz-Grafikobjekts (43).

9. Verfahren nach einem der Ansprüche 1 bis 8, ferner umfassend:
Erfassen einer Position und Orientierung der Anzeigevorrichtung (29);
wobei die Position und die Orientierung des Einführungsassistenz-Grafikobjekts (43) in der grafischen Darstellung (41, 47, 51, 57) ferner basierend auf der erfassten Position und Orientierung der Anzeigevorrichtung (29) bestimmt werden.

10. Verfahren nach einem der Ansprüche 1 bis 9,
wobei das Einführungsassistenz-Grafikobjekt (43) als Objekt der erweiterten Realität, als Objekt der virtuellen Realität und/oder in einem Bild (59) dargestellt wird.

11. Brachytherapieverfahren, umfassend:
das Verfahren nach einem Ansprüche 1 bis 10,
wobei vor dem Erfassen der Position und Orientierung der Applikatorführung (5) die Applikatorführung (5) in einen Gegenstand (31), insbesondere einen menschlichen oder tierischen Körper, eingeführt wird und
wobei bei dem Erfassen der Position und Orientierung der Applikatorführung (5) die Position und die Orientierung der in den Gegenstand (31) eingeführten Applikatorführung (5) erfasst werden.

12. Brachytherapieverfahren Anspruch 11, ferner umfassend:
Einführen des Applikators (3) in die Applikatorführung (5) unter Verwendung des dargestellten Einführungsassistenz-Grafikobjekts (43); und/oder
Erzeugen von Röntgenstrahlung im Inneren des in die Applikatorführung (5) eingeführten Applikators (3).

13. Brachytherapiesystem (23), umfassend:
einen Applikator (3);
eine Applikatorführung (5);
ein Navigationssystem (25);
eine Datenverarbeitungsvorrichtung (27); und
eine Anzeigevorrichtung (29),
wobei das Brachytherapiesystem (23) so konfiguriert ist, dass ein Verfahren gemäß einem der Ansprüche 1 bis 12 damit durchführbar ist.
